# FASCICULE DE BREVET EUROPEEN

(11) **EP 0 751 931 B1**
(45) Date de publication et mention de la délivrance du brevet: **01.12.1999**
(21) Numéro de dépôt: 95914412.2
(22) Date de dépôt: 24.03.1995
(51) Int. Cl.: C07C 263/20

(54) **OBTENTION DE 2.6-TOLUYLENE DIISOCYANATE A PARTIR D'UN MELANGE COMPRENANT DU 2.6-TOLUYLENE DIISOCYANATE ET DU 2.4-TOLUYLENE DIISOCYANATE**
GEWINNUNG VON 2.6- TOLUYLENDIISOCYANAT AUS EINE MISCHUNG VON 2.6- TOLUYLENDIISOCYANAT UND 2.4- TOLUYLENDIISOCYANAT
OBTAINING TOLUENE 2,6-DIISOCYANATE FROM A MIXTURE CONTAINING TOLUENE 2,6-DIISOCYANATE AND TOLUENE 2,4-DIISOCYANATE

(30) Priorité: 25.03.1994 FR 9403640
(43) Date de publication de la demande: 08.01.1997
(73) Titulaire: RHODIA CHIMIE, 92408 Courbevoie Cédex (FR)
(72) Inventeur: PARRON, Jean-Claude, F-69300 Caluir (FR); REVELANT, Denis, F-69740 Genas (FR); VACHET, François, F-69150 Decines (FR)
(74) Mandataire: Delenne, Marc
(86) Numéro de dépôt international: FR9500366
(87) Numéro de publication internationale: WO9526332

(56) Documents cités:
- US-A- 2 831 012
- US-A- 3 591 617

## Description

La présente invention a pour objet un procédé d'obtention de 2.6 toluylène diisocyanate à partir d'un mélange des isomères 2.4 et 2.6 du toluylène diisocyanate.

Les isomères 2.4 et 2.6 du toluylène diisocyanate (par la suite TDI) sont des composés très importants, à la base de l'élaboration des polyuréthannes dont les applications sont nombreuses. Ainsi ces polymères peuvent être utilisés pour fabriquer des mousses souples dont l'une des principales applications sont l'ameublement, la sellerie automobile. Ils peuvent de même être employés pour la fabrication d'élastomères de haute performances et entrent dans la composition de peintures mono ou bicomposants.

La plupart des applications industrielles du TDI mettent en oeuvre les isomères 2.4 et 2.6 en mélange. Le mélange le plus couramment utilisé comprend 80 % de l'isomère 2.4 et 20 % de l'isomère 2.6. L'emploi du mélange 65/35 en isomère 2.4 et 2.6 est aussi connu, bien que plus restreint, dans le domaine par exemple des mousses souples flexibles à haute portance.

Cependant, les polyuréthannes synthétisés à partir des isomères purs ont des propriétés particulièrement intéressantes, comparés à ceux obtenus à partir des mélange de ces isomères. Ainsi, le 2.6-TDI présente des propriétés remarquables quand il est employé comme composant dans la synthèse d'élastomères. En effet, dans le cas particulier des systèmes polyesters ou polycaprolactames couplés avec des diamines, le 2.6-TDI améliore fortement la résistance à la déchirure de même que la résistance à la propagation de la déchirure. En outre, la dureté et la résilience sont améliorées et le module est augmenté de 100 à 200 %. Dans le cas où l'on utilise des diols comme agents de couplage, la résistance à la déchirure et à la propagation de la déchirure sont aussi considérablement augmentées.

L'intérêt d'obtenir l'isomère 2.6-TDI pur est donc très important. Diverses voies sont connues pour accéder à ce produit. On peut citer notamment les techniques de séparation chromatographique, dans lesquelles le mélange est traité sur une colonne remplie de zéolite par exemple. Ces méthodes peuvent être mises en oeuvre directement sur les deux isomères du TDI mais elles peuvent de même l'être pour séparer les précurseurs de ces composés, comme par exemple les isomères 2.4 et 2.6 du dinitrotoluylène, ou du diaminotoluylène.

II y a cependant des inconvénients à procéder de la sorte. En effet, ces techniques obligent à travailler avec des quantités très diluées de mélange et nécessite l'emploi de solvant rigoureusement anhydre. Or la présence de traces d'eau est bien souvent inévitable et celles-ci réagissent avec les groupements isocyanates pour produire de l'urée. Ceci entraîne donc une baisse d'efficacité de la colonne, voire un bouchage de celle-ci. En outre, lorsque les précurseurs du TDI sont traités, il est nécessaire de disposer par la suite de moyens industriels indépendants permettant la transformation en isocyanate, de chacun des composés purifiés.

On connaît aussi, par le brevet américain US 2 831 012, un procédé de séparation de l'isomère 2.4 TDI d'un mélange comprenant les isomères 2.4 et 2.6, en faisant réagir l'isomère 2.4 avec de l'acide chlorhydrique pour donner le chlorure de carbamyle, qui est ensuite séparé du mélange à purifier. Le mélange résultant, après réaction et séparation ultérieure de l'isomère 2.4 ayant réagi, est purifié, par une méthode classique qui consiste à refroidir le mélange pour obtenir une cristallisation de l'isomère 2.6.

La présente invention a donc pour but de proposer un procédé d'obtention de l'isomère 2.6-TDI à partir d'un mélange d'isomères 2.4 et 2.6-TDI, dans lequel on soumet ledit mélange, comprenant au moins 50 % d'isomère 2.6-TDI, à au moins un cycle de cristallisation-fusion comprenant les étapes suivantes :
- on effectue une étape de cristallisation en diminuant la température jusqu'à une température voisine de celle à laquelle le mélange se trouve sous une forme solide, plus particulièrement jusqu'à une température voisine de -15 °C,
- on effectue un égouttage consistant à garder la température constante et à laisser le liquide s'écouler,
- on effectue une fusion du mélange en augmentant la température de la température voisine de celle à laquelle le mélange se trouve sous une forme solide, plus particulièrement une température voisine de -15 °C, jusqu'à la température de fusion de l'isomère 2.6.

Par ailleurs, le procédé selon l'invention présente l'avantage de donner des sous-produits valorisables notamment dans les applications classiques des isocyanates.

Elle a par ailleurs pour objet un procédé d'obtention d'isomère 2.6-TDI à partir d'un mélange d'isomères 2.4 et 2.6-TDI, dans lequel on met en contact ledit mélange, comprenant au moins 50 % d'isomère 2.4-TDI, avec un composé ou un catalyseur susceptible de transformer une partie de l'isomère 2.4-TDI présent et en ce que l'on sépare l'isomère 2.4 ayant réagi, puis on soumet le mélange résultant à au moins un cycle de cristallisation-fusion.

Le procédé selon l'invention présente l'avantage de donner des sous-produits valorisables notamment dans les applications classiques des isocyanates.

Mais d'autres avantages et caractéristiques de l'invention apparaîtront plus clairement à la lecture de la description et des exemples qui vont suivre.

Le mélange des isomères 2.4 et 2.6-TDI que l'on peut traiter conformément au procédé selon l'invention comprend d'une façon avantageuse, une proportion quelconque entre les deux isomères.

Selon une première variante du procédé de l'invention, le mélange traité comprend au moins 50 % d'isomère 2.6-TDI.

Dans ce cas, le mélange est soumis à un ou plusieurs cycles cristallisation-fusion, dont le processus sera décrit plus loin en détails.

Selon une seconde variante du procédé selon l'invention, le mélange à traiter comprend au moins 50 % de l'isomère 2.4-TDI.

Dans ce cas, préalablement aux cycles de cristallisation-fusion, on met le mélange à traiter en contact avec un composé ou un catalyseur susceptible de transformer une partie de l'isomère 2.4-TDI présent et en ce que l'on sépare l'isomère 2.4 ayant réagi.

Cette variante du procédé est particulièrement avantageuse en ce sens qu'elle permet le traitement de mélanges directement issus de la phosgénation des amines correspondantes ou encore de la réaction d'hydrogénation des dinitrotoluènes correspondants, suivie de la phosgénation des amines résultantes. Par exemple, les mélanges comprenant 80 % de 2.4-TDI et 20 % de 2.6-TDI, ou encore 65 % de 2.4-TDI et 35 % de 2.6-TDI conviennent à la mise en oeuvre de l'invention.

Ainsi, dans le cas de cette seconde variante, le procédé selon la présente invention peut être séparé en deux parties distinctes, la première ayant plus particulièrement trait à la transformation de l'isomère 2.4-TDI et la seconde à l'obtention de l'isomère 2.6-TDI, de façon avantageuse substantiellement pur.

La première partie du procédé tel que défini ci-dessus, consiste donc à transformer le 2.4-TDI de façon à pouvoir séparer le produit obtenu de l'isomère 2.6-TDI.

Etant donné la réactivité des deux isomères, l'isomère 2.4 sera transformé en priorité. Cependant, malgré la sélectivité importante des réactions mises en jeu vis-à-vis de l'isomère 2.4, on ne peut exclure qu'une partie, toutefois considérablement plus réduite, de l'isomère 2.6 réagisse aussi.

Pour des raisons de simplification, dans ce qui va suivre, il ne sera fait référence qu'à la transformation de l'isomère 2.4, sachant qu'une partie de l'isomère 2.6 peut avoir été transformée.

Deux types de réactions peuvent être convenablement mises en oeuvre pour transformer l'isomère 2.4.

Ainsi, on peut mettre en contact le mélange à traiter avec un catalyseur favorisant la réaction des groupements isocyanates sur eux-mêmes. De cette façon on obtient notamment des dimères, des trimères de l'isomère en question. Il est de même possible d'obtenir des composés du type carbodiimides.

Les catalyseurs susceptibles d'être utilisés sont bien connus de l'homme du métier. A titre d'exemples de catalyseurs de dimérisation ou trimérisation, on peut citer notamment les phosphines, les bases de Mannich, les composés basiques à base d'alcalin ou d'alcalino-terreux, comme par exemple l'acétate de sodium. A titre d'exemples de catalyseurs utilisables pour effectuer une carbodiimidification, on peut mentionner les oxydes de phospholine. Bien entendu, ces deux listes ne sont pas à considérer comme exhaustives.

Il est de même possible de mettre en contact le mélange à traiter avec un agent nucléophile.

Tous les composés de ce type conviennent à la mise en oeuvre de cette réaction. On peut notamment citer, sans toutefois s'y limiter, l'eau, les hydracides, tel que l'acide chlorhydrique principalement, les alcools aliphatiques ou aromatiques, comme les composés du type du phénol, substitués ou non par des radicaux hydrocarbonés, les acides carboxyliques, les esters maloniques, les amines ou les hydroxylamines, les oximes, les mercaptans.

Selon un mode particulier de réalisation de l'invention, on effectue cette transformation de telle sorte qu'une partie importante de l'isomère 2.4 soit transformée, de façon à limiter la transformation de l'isomère 2.6.

Selon un mode préféré, les catalyseurs et agents nucléophiles sont employés de telle façon qu'à l'issue de la réaction, la composition du mélange en isomères 2.4 et 2.6 libres se situe dans la nappe de cristallisation de l'isomère 2.6-TDI.

Plus particulièrement, la teneur du mélange en isomère 2.6-TDI libre est supérieure à 50 %.

Ainsi, dans le cas où l'on utilise un catalyseur, le degré d'avancement de la réaction est contrôlé notamment par dosage des groupements isocyanates libres restants. Lorsque le degré de transformation souhaité est atteint, la réaction est stoppée par toute méthode connue de l'homme du métier, selon la nature du catalyseur.

Selon un mode de réalisation préféré, la transformation de l'isomère 2.4 est menée de façon à obtenir un composé valorisable, notamment dans les domaines classiques d'application des isocyanates.

Les dimères, trimères et carbodiimides obtenus par l'action d'un catalyseur tels que ceux mentionnés auparavant sont des composés bien connus de l'homme du métier et couramment utilisés dans des applications telles que celles mentionnées auparavant.

L'utilisation d'agents nucléophiles tels que l'eau ou les amines primaires est avantageuse car elle conduit à la formation de groupements de type urée ; liaisons qui peuvent être transformées par la suite en groupements du type biuret, dont l'emploi dans la préparation de mousses souples notamment est bien connu.

L'action des hydracides, principalement l'acide chlorhydrique, est de même avantageuse car elle permet d'accéder à des chlorures de carbamyle, qui peuvent être décomposés à nouveau en isocyanates.

Par cette réaction de transformation de l'isomère 2.4-TDI, il est de même possible d'obtenir des composés hémi-bloqués de l'isomère 2.4 en utilisant notamment des agents nucléophiles du type des phénols, lactames, cétoximes. De tels composés peuvent être des intermédiaires d'un TDI totalement bloqué, par l'action ultérieure d'un agent de blocage, identique ou différent au premier agent employé. Les TDI totalement bloqués trouvent notamment une application dans le domaine des peintures.

Les composés hémi-bloqués peuvent de même, par réaction avec des polyols de bas poids moléculaire comme le glycérol, le triméthylol propane donner des agents de réticulation externes utilisés par exemple dans des peintures d'électrophorèse. Enfin , la réaction des composés hémi-bloqués avec des polyéthers ou des polyesters conduit à des prépolymères bloqués.

La réaction de transformation de l'isomère 2.4-TDI peut être réalisée indifféremment en présence ou non d'un solvant, choisi parmi des composés inertes vis-à-vis des divers constituants du mélange et dans les conditions de la réaction.

De préférence, les solvants utilisés doivent être anhydres.

Par ailleurs, mais sans que cela soit nécessaire, ledit solvant peut être choisi de telle sorte que le produit issu de la transformation de l'isomère 2.4-TDI ne soit pas soluble dans celui-ci.

Les solvants polaires ou apolaires conviennent particulièrement à la mise en oeuvre de la réaction. A titre d'exemple, on peut citer le chlorobenzène, l'orthodichlorobenzène, le xylène, le chloronaphtalène, le décahydronaphtalène, l'hexane, le tétrachlorure de carbone.

Il est à noter que la réaction de transformation peut être effectuée en une ou plusieurs étapes.

Ainsi, le renouvellement de cette étape peut être nécessaire si l'on n'a pas atteint de composition se situant dans la nappe de cristallisation du 2.6-TDI. Ceci est par exemple souhaitable lorsque le produit transformé augmente la viscosité du mélange total et de ce fait rend difficile la séparation ultérieure des sous-produits du mélange comprenant les isomères libres. Ceci permet aussi d'éviter d'employer de trop grandes quantités de solvant.

L'intérêt d'effectuer, en plusieurs fois, cette première partie du procédé selon l'invention est d'obtenir un mélange plus enrichi en isomère 2.6-TDI de façon à augmenter le rendement de la seconde étape du procédé.

La réaction est par ailleurs effectuée à une température dont le choix dépend de la composition du mélange des isomères 2.4 et 2.6-TDI, de la présence ou non d'un solvant, et de la sélectivité de la réaction de transformation vis-à-vis de l'isomère 2.4-TDI.

Ainsi, température de la réaction est relativement basse, pour favoriser la transformation sélective de l'isomère 2.4-TDI, mais elle n'est pas, de préférence, inférieure au point de cristallisation du mélange à traiter. Cette température peut en outre varier au cours de la réaction, mais l'homme du métier est à même de la modifier.

Dans le cas particulier d'un mélange comprenant 65 % d'isomère 2.4-TDI et 35 % d'isomère 2.6-TDI, la température est voisine de 10°C.

La mise en contact du mélange, éventuellement en présence du solvant précité, avec le catalyseur ou l'agent nucléophile, est réalisée sous agitation.

Une fois la réaction terminée, on sépare le produit formé du mélange à présent enrichi en isomère 2.6-TDI.

Cette séparation peut être réalisée par tout moyen connu, comme la filtration, la décantation, l'extraction, la distillation.

Si un solvant a été utilisé pendant la réaction, celui-ci doit être éliminé avant le traitement du mélange enrichi en isomère 2.6-TDI. Généralement, cette opération est effectuée par distillation.

La seconde partie du procédé selon l'invention a pour objet la séparation de l'isomère 2.6-TDI du mélange enrichi en cet isomère, résultant des étapes précédentes.

L'opération , appelée aussi raffinage, correspond à un ou plusieurs cycles de cristallisation-fusion du mélange.

L'étape de cristallisation du cycle consiste à refroidir progressivement le mélange à traiter de la température ambiante jusqu'à une température voisine de celle à laquelle le mélange se trouve sous une forme solide, que l'on appellera température du point eutectique. Une telle température se situe vers -15 °C et plus particulièrement voisine de -13°C.

De préférence, l'étape de cristallisation est menée en refroidissant le mélange jusqu'à une température dont l'écart avec la température du point eutectique est compris entre 2 et 5°C.

La vitesse de diminution de la température est comprise entre -0,5 et -4°C/heure. Cette vitesse peut être conservée constante pendant toute l'opération de baisse en température. Cependant, selon une variante préférée, on procède par paliers en augmentant la vitesse à mesure que la température du milieu est abaissée.

On procède ensuite à une étape d'égouttage qui consiste à garder la température constante et à laisser le liquide, riche en isomère 2.4-TDI et en d'autres impuretés si elles existent, s'écouler. Ce résidu liquide est donc extrait de l'appareil.

Il est à noter que ce résidu peut être traité par une réaction chimique telle que décrite auparavant, afin de l'enrichir en isomère 2.6-TDI ; mélange qui pourra ensuite être traité conjointement avec un nouveau mélange à traiter selon les cycles de cristallisation - fusion.

Généralement, la durée de cette étape est comprise entre 30 minutes et 3 heures.

D'une manière avantageuse et afin de minimiser la durée de ce pallier, on peut effectuer cette opération sous une légère surpression d'un gaz anhydre et neutre, tel que l'azote ou les gaz rares par exemple.

La seconde étape du cycle est réalisée en augmentant progressivement la température du mélange jusqu'à la température ambiante. De préférence, on augmente la température du mélange jusqu'à la température de fusion du 2.6-TDI.

La vitesse de d'augmentation de la température est comprise entre 0,5 et 8°C/heure. Là encore, cette vitesse peut être conservée constante pendant toute l'opération de remontée de la température. Cependant, selon une variante préférée, on procède par paliers en diminuant la vitesse à mesure que la température du milieu est plus élevée.

Selon une variante préférée de l'invention, l'étape de fusion est réalisée en deux parties. La première consiste en un réchauffage lent de la masse cristallisée restant dans l'appareil après évacuation du résidu liquide précité. De cette façon, on élimine la fraction restante de ce résidu liquide qui se trouve dans les cristaux, par porosité.

La fraction liquide qui s'écoule alors présente une pureté moyenne comprise entre celle du résidu précité et celle du produit final désiré.

Il est à noter que ce liquide peut être valorisé en tant que qualité de produit intermédiaire, ou recyclé dans un cycle ultérieur de cristallisation -fusion, conjointement avec un nouveau mélange à traiter, ou encore joint au résidu liquide précité pour être ensuite traité par réaction chimique conformément à ce qui a été explicité auparavant.

La seconde partie de cette étape consiste en la fusion rapide des cristaux présentant la pureté attendue.

D'une manière classique pour l'homme du métier, on peut contrôler la pureté de la fraction liquide qui s'écoule par tout moyen, comme par exemple l'analyse de la composition de celle-ci ou encore plus simplement en contrôlant la température de ladite fraction liquide. En effet, la température est fonction de la composition de la fraction liquide.

La durée de chacune de ces étapes est comprise entre 5 et 20 heures.

Selon une variante particulièrement avantageuse de la présente invention, et plus spécialement lorsque l'on met en oeuvre un cycle ultérieur de cristallisation - fusion sur un nouveau mélange à traiter, on effectue une opération de glaçage avant de commencer ce nouveau cycle.

Celle-ci consiste à refroidir l'appareillage vide, qui comprend sur ses parois, un film enrichi en isomère 2.6-TDI, provenant d'une opération précédente notamment, à une température supérieure de 2 à 3°C par rapport à la température du point eutectique, tel que défini précédemment. En outre cette température est plus particulièrement inférieure de 6 à 10°C par rapport à la température à laquelle apparaissent les premiers cristaux du mélange considéré lorsque ledit mélange est à l'équilibre thermodynamique. Cette température est fonction de la composition du mélange considéré et peut être déterminée par tout moyen connu de l'homme du métier.

La diminution de température est effectuée très rapidement, plus particulièrement avec une vitesse de l'ordre de -20°C/h.

On maintient ensuite cette température pendant une durée de l'ordre de quelques dizaines de minutes. Puis la température est rapidement remontée, par exemple avec une vitesse de l'ordre de 20°C/h, jusqu'à une température inférieure de 2 à 6°C par rapport à celle à laquelle apparaissent les premiers cristaux du mélange considéré lorsque le mélange est à l'équilibre thermodynamique.

Le mélange à traiter est alors introduit dans l'appareillage.

On laisse ensuite la température du mélange se stabiliser pendant une durée d'environ 1 à 2 heures, avant de commencer le cycle cristallisation - fusion décrit précédemment.

Le ou les cycles de cristallisation-fusion sont réalisés sans agitation.

Les cycles de cristallisation - fusion selon l'invention peuvent être mis en oeuvre dans tout type d'appareillage connu.

On peut ainsi procéder dans un appareillage dit statique. Dans ce cas, le liquide à traiter est introduit dans une capacité contenant un échangeur thermique de grande surface par rapport au volume de la capacité, munie de moyen de contrôle de la température des parois sur lesquelles se déposent les cristaux purifiés.

On peut de même effectuer les cycles dans un appareillage dit dynamique. Dans ce cas de figure, on fait ruisseler ou circuler le liquide à traiter dans des tubes verticaux, le fluide caloporteur ruisselant ou circulant de l'autre côté du tube. Le mélange à traiter effectue ainsi un grand nombres de passages sur la paroi froide sur laquelle se déposent les cristaux purifiés. Cet appareil permet de raccourcir la durée des cycles de cristallisation - fusion mais il est souvent nécessaire pour augmenter le rendement d'effectuer plusieurs enchaînements de cristallisation - fusion sur les produits résiduels et purifiés issus de l'étape précédente.

Selon un mode particulier de réalisation de l'invention, le ou les cycles cristallisation-fusion sont mis en oeuvre sous une atmosphère dépourvue d'eau.

De préférence, l'atmosphère sous laquelle le cycle est effectué est choisie parmi les gaz rares secs, comme l'argon, ou encore sous azote sec.

La pression sous laquelle est réalisé le cycle cristallisation-fusion est en général voisine de la pression atmosphérique ou légèrement supérieure.

Pendant l'étape de fusion, on sépare la phase liquide des cristaux, qui comprennent principalement l'isomère 2.6-TDI.

Il est à noter que l'on peut effectuer de nouveaux cycles de cristallisation - fusion sur des cristaux enrichis en isomère 2.6-TDI, obtenus après l'étape précédente de raffinage, afin d'en améliorer la pureté. Il est à noter que le recyclage dans le procédé selon l'invention, des fractions liquides de pureté non suffisante permet d'augmenter le rendement de ce demier.

Le procédé selon l'invention permet donc de récupérer des mélanges enrichis en isomère 2.6-TDI, dont la composition en cet isomère est supérieure à celle du mélange initial. D'une façon avantageuse, le procédé selon l'invention permet d'obtenir l'isomère 2.6-TDI avec une pureté d'au moins 90 % et pouvant aller jusqu'à 99%.

Des exemples concrets mais non limitatifs de l'invention vont maintenant être présentés.

### EXEMPLE 1

Cet exemple illustre la première partie du procédé selon l'invention.

On utilise un réacteur en verre de 2 litres :
- muni d'une double enveloppe dans laquelle circule un fluide frigoporteur alimenté par un bain thermostaté type LAUDA,
- équipé d'une agitation efficace type turbine Rushton,
- d'un réfrigérant vertical,
- et d'une alimentation en HCI anhydre type plongeur,

On charge dans ce réacteur :

| | |
|---|---|
| O- dichlorobenzène | 500 g |
| Toluylène di isocyanate 65/35 | 500 g |

(soit : 1,87 mole de 2.4 TDI et 1 mole de 2.6 TDI)

Après refroidissement du mélange réactionnel à 10°C, on introduit sous agitation 34 g d'acide chlorhydrique gazeux (0,931 mole) en 1 heure. La température du mélange réactionnel est maintenue entre 10 et 12°C.

Il se forme rapidement un mélange biphasique solide-liquide qui s'épaissit en fin d'addition de l'acide chlorhydrique.

Le mélange réactionnel est filtré. Le solide est lavé à l'hexane et séché à température ambiante sous vide.

On obtient ainsi :
- une phase solide constituée de :
   . 189,5 g de 4 monochlorure de carbamyle (0,90 mole de TDI)
   . 4,2 g de 2 monochlorure de carbamyle (0,02 mole de TDI)
   Le résultat est déterminé par spectrométrie IR, après dissociation des chlorures de carbamyle, sur les toluylène diisocyanate correspondants.
- une phase liquide ayant la composition suivante :
   . 0- dichlorobenzène 480 g
   . 2.4 Toluylène di isocyanate 168,4 g (0,97 mole)
   . 2.6 Toluylène di isocyanate 170,5 g (0,98 mole)

La composition du mélange des isomères du TDI est 50/50, déterminée par spectrométrie IR.

La même opération est répétée avec la phase liquide obtenue précédemment à la quelle on ajoute 34 g d'acide chlorhydrique en maintenant la température aux alentours de 10°C.

Après filtration, on obtient :
- une phase solide constituée de :
   . 153,2 g de 4 monochlorure de carbamyle de TDI (0,73 mole)
   . 6,3 g de monochlorure de carbamyle de TDI (0,03)
- une phase liquide ayant la composition suivante :
   . O-dichlorobenzène 460 g
   . 2.4 TDI 41,8 g (0,24 mole)
   . 2.6 TDI 165,3 g (0,95 mole)

La composition du mélange est de 20/80 en isomères 2.4/2.6

Après élimination par distillation de l'o-dichlorobenzène, on obtient 205 g de mélange 2,4/2,6 TDI comprenant 80 % en isomère 2.6 du TDI.
On obtient ainsi un mélange des 2 isomères du TDI se situant dans la nappe de cristallisation de l'isomère 2.6.

Remarque : Le mélange des chlorures de carbamyle peut subir une déshydrochloration pour donner un mélange 97/3 de 2.4 TDI/2.6 TDI.

### EXEMPLE 2

Cet exemple illustre la seconde partie du procédé selon l'invention.

On utilise un raffinoir statique de 300 ml de capacité utile, constitué par un cylindre en acier inoxydable double enveloppé de diamètre 50 mm et de hauteur 40 mm :
- équipé d'une vanne de fond automatique et double enveloppée ayant son propre circuit thermostatique,
- pourvu d'un collecteur automatisé sur balance,
- alimenté par un circuit programmable de chauffage ou de refroidissement,
- et piloté par un automate programmable muni d'une centrale d'acquisition de données (température du produit et du fluide caloporteur, masses des fractions et temps).

L'appareillage fonctionne sous légère surpression d'argon.
On charge dans ce raffinoir 154 g du mélange obtenu dans l'exemple 1.

La cristallisation du mélange isomérique s'effectue, après un amorçage de la cristallisation à -4,7°C, entre 3.7°C et -10,8°C en 14 heures, y compris la stabilisation finale en température et l'élimination du résidu liquide.
La fusion, comprenant le réchauffage lent, est effectuée en 15 heures entre -10,8°C et 19,2°C.

On isole 7 fractions au cours de la fusion comprenant une teneur croissante en isomère 2.6-TDI (déterminée par analyse).

On constate que, par réunion de certaines fractions, 55 % de la charge récupérée présente un titre en 2.6-TDI voisin de 95 % ou que par réunion d'autres fractions, 20 % de la charge récupérée présente une teneur de 99 % en 2.6-TDI.

### EXEMPLE 3

Dans un réacteur en verre de 1 litre équipé :
- d'une agitation efficace type cadre,
- d'une ampoule de coulée de 250 ml,
- d'un condenseur vertical,
- d'un système de réfrigération,
- et respirant sous atmosphère anhydre.

On charge :
- Toluylène di isocyanate 65/35 870 g
   (soit 3,25 moles de 2.4 et 1,75 mole de 2.6 TDI)

Après refroidissement du mélange de TDI à une température entre 8°C et 10°C, on coule en 1h30, en maintenant à la température à cette valeur, 216 g de méthyl éthyl cétoxime.
On obtient un liquide homogène visqueux qui est soumis à la distillation sous vide.

On obtient ainsi :
- 384 g de distillat composé de 203,5 g de 2.4-TDI (1,17 mole) et de 180,5 g de 2.6-TDI (1,04 mole),

La composition de ce mélange correspond à 53 % de 2.4-TDI et 47 % de 2.6-TDI.
- 674 g de liquide visqueux ayant la composition suivante :
   . TDI libre (2.4 + 2.6) 99,8 g (0,57 mole)
   . TDI hémi-bloqué 440,89 (1,69 mole)
   . TDI totalement bloqué 111,2 g (0,32 mole)
   . lourds 22,2 g
   (résultats obtenus par examen par chromatographie d'exclusion-diffusion/IR transformée de Fourier)

La même opération est répétée avec le distillat obtenu précédemment auquel on ajoute 87 g de méthyl éthyl cétoxime (1 mole) en 40 minutes à une température voisine de 10°C.
On obtient un liquide homogène visqueux qui est soumis à la distillation.

On obtient ainsi :
- 193 g de distillat composé de 75,3 g de TDI 2.4 (0,43 mole) et 117,7 g de TDI 2.6 (0,68 mole), soit un mélange de T 39.
- 264 g d'un mélange de composés visqueux ayant la composition suivante :
   . TDI libre (2.4 + 2.6) 30,1 g (0,17 mole)
   . TDI hémi-bloqué 146,5 g (0,56 mole)
   . TDI totalement bloqué 49,6 g (0,14 mole)
   . lourds 37,8 g

Le rendement pour les 2 étapes par rapport au 2.6 TDI 2.6 récupéré / 2.6 engagé est de (0,68 X 100) / 1,75 = 39 %.

On obtient un mélange des 2 isomères du TDI se situant dans la nappe de cristallisation de l'isomère 2.6. Le cycle cristallisation fusion selon l'exemple 2 a été reproduit et permet d'obtenir l'isomère 2.6 pur à 99 %.

### EXEMPLE 4

Cet exemple illustre la seconde partie du procédé selon l'invention.

On utilise un raffinoir statique de 4 litres de capacité utile, constitué par un cylindre en acier inoxydable double enveloppé de diamètre 55 mm et de hauteur 1600 mm :
- équipé d'une vanne de fond automatique et double enveloppée ayant son propre circuit thermostatique,
- pourvu d'un collecteur automatisé sur balance,
- alimenté par un circuit programmable de chauffage ou de refroidissement,
- et piloté par un automate programmable muni d'une centrale d'acquisition de données (température du produit et du fluide caloporteur, masses des fractions et temps).

L'appareillage fonctionne sous légère surpression d'argon.
On charge dans ce raffinoir 4830 g d'un mélange contenant 78,6 % de 2.6-TDI.

On effectue un glaçage de la paroi humide de l'appareil vide à une température voisine de -4°C.
Le mélange à traiter est alors chargé dans l'appareil.

La phase de cristallisation est effectuée entre 6,1 et - 10,5°C en 10,5 heures, avec une période de 2 heures pour stabiliser la température du mélange à - 10,5°C.
Le liquide résiduel est alors éliminé. Il représente une pureté de 47,8 % en 2.6-TDI.

On réchauffe la mélange en 12 heures en augmentant la température de - 10,5 à 16,7°C.
La fraction liquide récupérée présente une pureté de 76,6 % en 2.6-TDI.

La fusion finale a lieu en 1 heure en augmentant la température de 16,7 à 21°C.

On récupère une masse représentant 34 % de la charge initiale avec une pureté de 99,2% en 2.6-TDI.

## Revendications

1. Procédé d'obtention d'isomère 2.6 de toluylène diisocyanate à partir d'un mélange d'isomères 2.4 et 2.6 du toluylène diisocyanate, caractérisé en ce que l'on soumet ledit mélange, comprenant au moins 50 % de 2.6-toluylène diisocyanate, à au moins un cycle de cristallisation-fusion comprenant les étapes suivantes :
- on effectue une étape de cristallisation en diminuant la température jusqu'à une température voisine de celle à laquelle le mélange se trouve sous une forme solide, plus particulièrement jusqu'à une température voisine de -15 °C,
- on effectue un égouttage consistant à garder la température constante et à laisser le liquide s'écouler,
- on effectue une fusion du mélange en augmentant la température de la température voisine de celle à laquelle le mélange se trouve sous une forme solide, plus particulièrement une température voisine de -15 °C, jusqu'à la température de fusion de l'isomère 2.6.

2. Procédé d'obtention d'isomère 2.6 de toluylène diisocyanate à partir d'un mélange d'isomères 2.4 et 2.6 du toluylène diisocyanate, caractérisé en ce que l'on met en contact ledit mélange, comprenant au moins 50 % de 2.4-toluylène diisocyanate, avec un composé ou un catalyseur susceptible de transformer une partie de l'isomère 2.4-toluylène diisocyanate présent et en ce que l'on sépare l'isomère 2.4 ayant réagi, puis on soumet le mélange résultant à au moins un cycle de cristallisation-fusion, selon la revendication 1.

3. Procédé selon la revendication précédente, caractérisé en ce que le composé est choisi parmi les agents nucléophiles du type de l'eau, des hydracides, comme l'acide chlorhydrique, des alcools aliphatiques ou aromatiques, comme les composés du type du phénol, substitués ou non par des radicaux hydrocarbonés, les acides carboxyliques, les esters maloniques, les amines ou les hydroxylamines, les oximes.

4. Procédé selon la revendication 2, caractérisé en ce que le catalyseur est choisi parmi les phosphines, les bases de Mannich, les composés basiques à base d'alcalin ou d'alcalino-terreux, les oxydes de phospholine.

5. Procédé selon l'une quelconque des revendications 2 à 4, caractérisé en ce que l'on met en contact le mélange de départ en présence d'un solvant polaire ou apolaire.

6. Procédé selon la revendication précédente, caractérisé en ce qu'avant d'effectuer le premier cycle cristallisation-fusion et après l'élimination de l'isomère 2.4 ayant réagi, on élimine le solvant.

7. Procédé selon l'une quelconque des revendications précédentes, caractérisé en ce que le cycle cristallisation-fusion est mis en oeuvre sous une atmosphère dépourvue d'eau.

8. Procédé selon l'une quelconque des revendications précédentes, caractérisé en ce que l'étape de cristallisation est effectuée en diminuant la température jusqu'à une température dont l'écart avec celle à laquelle le mélange se trouve sous une forme solide est compris entre 2 et 5°C.

9. Procédé selon la revendication précédente, caractérisé en ce que préalablement à l'étape de cristallisation, on effectue une opération de glaçage à une température supérieure de 2 à 3°C par rapport à la température à laquelle le mélange se trouve sous une forme solide, et inférieure de 6 à 10°C par rapport à la température à laquelle apparaissent les premiers cristaux du mélange lorsque celui-ci est à l'équilibre thermodynamique.

10. Procédé selon l'une quelconque des revendications précédentes, caractérisé en ce que la vitesse de diminution de la température dans l'étape de cristallisation est comprise entre -0,5 et -4 °C/heure.

11. Procédé selon l'une quelconque des revendications précédentes, caractérisé en ce que la vitesse d'augmentation de la température dans l'étape de fusion est comprise entre 0,5 et 8 °C/heure.

## Patentansprüche

1. Verfahren zur Gewinnung des 2,6-Isomers von Toluylendiisocyanat ausgehend von einer Mischung der 2,4- und 2,6-Isomere von Toluylendiisocyanat, dadurch gekennzeichnet, daß man die Mischung, die mindestens 50% 2,6-Toluylendiisocyanat enthält, mindestens einem Kristallisations-Schmelz-Zyklus unterwirft, der die folgenden Schritte umfaßt:
- man bewirkt einen Kristallisationsschritt, indem man die Temperatur bis zu einer Temperatur nahe jener, bei der die Mischung sich in einer festen Form befindet, insbesondere bis zu einer Temperatur nahe -15°C, verringert,
- man bewirkt ein Abtropfenlassen, das daraus besteht, die Temperatur konstant zu halten und die Flüssigkeit ablaufen zu lassen,
- man bewirkt ein Schmelzen der Mischung, indem man die Temperatur von der Temperatur nahe jener, bei der die Mischung sich in einer festen Form befindet, insbesondere einer Temperatur nahe -15°C, bis zu der Schmelztemperatur des 2,6-Isomers erhöht.

2. Verfahren zur Gewinnung des 2,6-Isomers von Toluylendiisocyanat ausgehend von einer Mischung der 2,4- und 2,6-Isomere von Toluylendiisocyanat, dadurch gekennzeichnet, daß man die Mischung, die mindestens 50% 2,4-Toluylendiisocyanat enthalt, mít einer Verbindung oder einem Katalysator in Berührung bringt, die bzw. der in der Lage ist, einen Teil des vorliegenden 2,4-Toluylendiisocyanat-Isomers umzuwandeln, und daß man das 2,4-Isomer, das reagiert hat, abtrennt, dann die resultierende Mischung mindestens einem Kristallisations-Schmelz-Zyklus nach Anspruch 1 unterwirft.

3. Verfahren nach dem vorangegangenen Anspruch, dadurch gekennzeichnet, daß die Verbindung ausgewählt wird unter den nukleophilen Mitteln vom Typ Wasser, der Wasserstoffsäuren, wie Salzsäure, der aliphatischen oder aromatischen Alkohole, wie den gegebenenfalls durch kohlenwasserstoffhaltige Reste substituierten Verbindungen des Phenoltyps, der Carbonsäuren, der Malonsäureester, der Amine oder der Hydroxylamine, der Oxime.

4. Verfahren nach Anspruch 2, dadurch gekennzeichnet, daß der Katalysator ausgewählt wird unter den Phosphinen, den Mannich-Basen, den basischen Verbindungen auf Basis von Alkali- oder Erdalkalimetallen, den Phospholinoxiden.

5. Verfahren nach einem der Ansprüche 2 bis 4, dadurch gekennzeichnet, daß man die Ausgangsmischung in Anwesenheit eines polaren oder apolaren Lösemittels in Berührung bringt.

6. Verfahren nach dem vorangegangenen Anspruch, dadurch gekennzeichnet, daß man vor dem Ausführen des ersten Kristallisations-Schmelz-Zyklus und nach dem Entfernen des 2,4-Isomers, das reagiert hat, das Lösemittel entfernt.

7. Verfahren nach einem der vorangegangenen Ansprüche, dadurch gekennzeichnet, daß der Kristallisations-Schmelz-Zyklus unter einer wasserfreien Atmosphäre ausgeführt wird.

8. Verfahren nach einem der vorangegangenen Ansprüche, dadurch gekennzeichnet, daß der Kristallisationsschritt ausgeführt wird, indem die Temperatur bis auf eine Temperatur verringert wird, deren Abstand zu jener, bei der sich die Mischung in einer festen Form befindet, zwischen 2 und 5°C beträgt.

9. Verfahren nach dem vorangegangenen Anspruch, dadurch gekennzeichnet, daß man vor dem Kristallisationsschritt einen Glasiervorgang bei einer Temperatur 2 bis 3°C höher als die Temperatur, bei der sich die Mischung in einer festen Form befindet, und 6 bis 10°C unter der Temperatur, bei der die ersten Kristalle der Mischung erscheinen, wenn jene sich im thermodynamischen Gleichgewicht befindet, ausführt.

10. Verfahren nach einem der vorangegangenen Ansprüche, dadurch gekennzeichnet, daß die Geschwindigkeit der Temperaturverringerung in dem Kristallisationsschritt zwischen -0,5 und -4°C/h liegt.

11. Verfahren nach einem der vorangegangenen Ansprüche, dadurch gekennzeichnet, daß die Geschwindigkeit der Temperaturerhöhung in dem Schmelzschritt zwischen 0,5 und 8°C/h liegt.

## Claims

1. Process for the production of a 2,6-toluylene diisocyanate isomer from a mixture of 2,4- and 2,6-isomers of toluylene diisocyanate, characterized in that the said mixture, comprising at least 50% 2,6-toluylene diisocyanate, is subjected to at least one cycle of crystallization-melting, comprising the following steps:
- a crystallization step is carried out by lowering the temperature to a temperature close to that at which the mixture is in a solid form, more particularly to a temperature in the region of -15°C,
- drainage is carried out, which consists in keeping the temperature constant and in allowing the liquid to flow,
- the mixture is melted by raising the temperature from the temperature close to that at which the mixture is in a solid form, more particularly a temperature of -15°C, up to the melting point of the 2,6- isomer.

2. Process for the production of the 2,6-toluylene diisocyanate isomer from a mixture of 2,4- and 2,6- isomers of toluylene diisocyanate, characterized in that the said mixture, comprising at least 50% 2,4-toluylene diisocyanate, is placed in contact with a compound or a catalyst which is capable of converting part of the 2,4-toluylene diisocyanate isomer present, and in that the 2,4- isomer which has reacted is separated out, then the resulting mixture is subjected to at least one cycle of crystallization-melting, according to Claim 1.

3. Process according to the preceding claim, characterized in that the compound is chosen from nucleophilic agents of the type such as water, hydracids, for instance hydrochloric acid, aliphatic or aromatic alcohols, for instance compounds of the phenol type which may or may not have hydrocarbon radical substituents, carboxylic acids, malonic esters, amines or hydroxylamines, and oximes.

4. Process according to Claim 2, characterized in that the catalyst is chosen from phosphines, Mannich bases, basic compounds based on alkali metal or on alkaline-earth metal, and phospholine oxides.

5. Process according to any one of Claims 2 to 4, characterized in that the starting mixture is placed in contact in the presence of a polar or apolar solvent.

6. Process according to the preceding claim, characterized in that before carrying out the first crystallization-melting cycle and after elimination of the 2,4- isomer which has reacted, the solvent is removed.

7. Process according to any one of the preceding claims, characterized in that the crystallization-melting cycle is carried out under a water-free atmosphere.

8. Process according to any one of the preceding claims, characterized in that the crystallization step is carried out by lowering the temperature to a temperature at which the difference from the temperature at which the mixture is in a solid form is between 2 and 5°C.

9. Process according to the preceding claim, characterized in that prior to the crystallization step, a chilling step is carried out at a temperature 2 to 3°C higher than the temperature at which the mixture is in a solid form, and 6 to 10°C lower than the temperature at which the first crystals of the mixture appear when it is at thermodynamic equilibrium.

10. Process according to any one of the preceding claims, characterized in that the rate of decrease of temperature in the crystallization step is between -0.5 and -4°C/hour.

11. Process according to any one of the preceding claims, characterized in that the rate of increase of temperature in the melting step is between 0.5 and 8°C/hour.
